# EUROPEAN PATENT SPECIFICATION

(11) **EP 3 240 907 B1**
(45) Date of publication and mention of the grant of the patent: **18.07.2018**
(21) Application number: 16712511.1
(22) Date of filing: 24.02.2016
(51) Int. Cl.: C12Q 1/04, C12Q 1/06, C12Q 1/58, G01N 31/22

(54) **ULTRA-RAPID DETECTION OF HELICOBACTER PYLORI IN GASTRIC MUCOSAL BIOPSIES**
ULTRASCHNELLE DETEKTION VON HELICOBACTER PYLORI IN MAGENSCHLEIMHAUTBIOPSIEN
DÉTECTION ULTRA-RAPIDE D'HELICOBACTER PYLORI DANS DES BIOPSIES DE MUQUEUSES GASTRIQUES

(30) Priority: 03.03.2015 IT FR20150002
(43) Date of publication of application: 08.11.2017
(73) Proprietor: Cagnoni, Marco, 00162 Roma (IT)
(72) Inventor: IOVINE, Valentina, 00162 Roma (IT); CAGNONI, Marco, I-00162 Roma (IT)
(74) Representative: Sarpi, Maurizio
(86) International application number: PCT/IB2016/051015
(87) International publication number: WO 2016/139558

(56) References cited:
- WO-A2-2011/064740
- CN-A- 103 757 088
- US-A- 5 702 911
- Biohit: "Helicobacter Pylori UFT300 Quick Test / Biohit HealthCare", , 10 November 2014 (2014-11-10), XP055236461, Retrieved from the Internet: URL:http://web.archive.org/web/20141110071 518/http://www.biohithealthcare.com/produc ts/diagnostics-tests/products/28/helicobac ter-pylori-quick-test-uft300 [retrieved on 2015-12-15]
- D. VAIRA ET AL: "Accuracy of a new ultra fast rapid urease test to diagnose Helicobacter pylori infection in 1000 consecutive dyspeptic patients", ALIMENTARY PHARMACOLOGY & THERAPEUTICS., 5 November 2009 (2009-11-05), XP055236245, GB ISSN: 0269-2813, DOI: 10.1111/j.1365-2036.2009.04196.x
- A. S. ARVIND: "ONE-MINUTE ENDOSCOPY ROOM TEST FOR CAMPYLOBACTER PYLORI", THE LANCET, vol. 331, no. 8587, 1 March 1988 (1988-03-01), page 704, XP055236440, GB ISSN: 0140-6736
- GOH KHEAN-LEE ET AL: "HUITAI rapid urease test: a new ultra-rapid biopsy urease test for the diagnosis of Helicobacter pylori infection.", JOURNAL OF DIGESTIVE DISEASES AUG 2007, vol. 8, no. 3, August 2007 (2007-08), pages 139-142, XP009187710, ISSN: 1751-2972
- BHASIN D K ET AL: "Relative merits of various rapid biopsy urease tests for diagnosis of Helicobacter pylori (Campylobacter pylori).", THE JOURNAL OF THE ASSOCIATION OF PHYSICIANS OF INDIA SEP 1990, vol. 38 Suppl 1, September 1990 (1990-09), pages 689-691, XP009187730, ISSN: 0004-5772

## Description

### Field of the invention

The present invention relates to a diagnostic composition in the form of aqueous solution for detecting very quickly the presence of *Helicobacter Pylori* in biopsy samples withdrawn during endoscopy analysis, and for evaluating the bacterial load thereof.

### Background of the invention

Some gastro-intestinal diseases, such as gastric or duodenal ulcers, and especially active chronic gastritis, are strictly associated to the partial or diffuse colonization of the gastric mucosa by *Helicobacter Pylori.* This bacterium, also known as *Campylobacter Pylori,* degrades urea by the urease enzyme. The *Helicobacter Pylori* presence in biopsy fragments of human gastric mucosa has already been recognized for several decades (Freedberg A.S. and Barron L.E. The presence of spirochetes in human gastric mucosa, 1940, Am. J. Dig. Dis., 7: 443-5; Marshall B.J. Unidentified Curved bacilli on Gastric epithelium in active chronic gastritis, 1983, Lancet, 1: 1273-5), and its causal correlation with type B chronic gastritis is widely documented (Rauws et al. 1988, Gastroenterology, 94: 33-40), as well as its involvement in the pathogenesis of peptic ulcer (Peterson W.L. Helicobacter pylori and peptic ulcer disease, 1991, New Eng. J. Med., 324: 1042- 8). *Helicobacter Pylori* is also considered a risk factor for duodenal ulcer recurrence (Coghlan J.G. et al. Campylobacter pylori and recurrence of duodenal ulcers at 12-month follow-up study. Lancet 1987; 2: 1109-1111) .

Consequently, the eradication of the bacterium causes histological improvement of gastritis, increases the likelihood of ulcerative healing and is effective in preventing recurrence.

The best method to diagnose the presence of *H. Pylori* in the gastric mucosa biopsies is consisting in the combination of histological and bacterial culture observations (Peterson W.L., Helicobacter pylori and Peptic Ulcer Disease, 1991 N. Engl. J. Med., 324: 1043-1048), although, in some studies, the value of the latter has been widely questioned (Martinez E., Marcos, A., Helicobacter pylori and peptic ulcer disease, 1991, Lancet, 325: 737).

The presence of *H. Pylori* can also be determined by searching for blood antibodies. However, this test can remain positive for several months after bacteria eradication, or be falsely positive in 10-15% of cases for cross-reaction with other antibodies.

Another method for determining the presence of *H. Pylori* includes Breath Test C13, which is expensive both for the isotopic material and the necessary equipment.

A valid alternative test uses the specific bacteria urease activity. To this end, a mucosal biopsy is incubated in a medium containing urea and a pH sensitive colour indicator (Owen R. J. et al., Rapid urea hydrolysis by gastric campilobacters, 1985, Lancet, 1: 111). Bacteria urease in the sample releases ammonia through hydrolysis of urea and when the amount of produced urea is sufficient to raise the pH value of the medium, the indicator changes colour. Urease is not produced by the human body, in fact, only occasionally biopsy fragments may contain urease derived from other bacteria, however, other bacteria strains, such as Proteus, Pseudomonas and *E. coli,* contain urease at much lower level (Kasper G., Dickgiesser N. Isolation from gastric epithelium of Campylobacter-like bacteria that are distinct from Campylobacter pyloridis, 1985 Lancet 8420: 111-112). Consistently to this mechanism, various diagnostic tests on mucosal biopsies have been developed. These include the technical solutions claimed by Marshall (U.S. Pat. No. 4,748,113, May 31, 1988) and Jackson (U.S. Pat. No. 5,439,801 Aug. 8, 1995).

Both describe a test composition containing urea producing ammonia by the *H. Pylori* urease enzyme. The reaction produces an increase of the pH of the medium which is relieved through the indicator colour change. In the Marshall test, the incubation medium is a gel containing phenol red as ph indicator, which turns from yellow to red when the ammonia released from the urea increases the pH to at least 6.9. The reaction system is buffered so that the fragments contaminated from blood or intestinal fluids do not raise the pH value causing a false positive result. The test requires an incubation time of 3 hours at 30-40 °C and up to 21 additional hours at room temperature. About 75% of *H. pylori* infected biopsies shows positive result in 20 minutes and 90% are positive in 3 hours. However, in order to verify the negative results 24 hours are required.

In Jackson tests, also performed in agar-gel, two colour indicators are used (methyl red and bromothymol blue) along with preservatives (paraben-methyl and propyl) in the absence of buffer systems. Over 95% of tests are positive within 20 minutes, some tests can take an hour, but all become positive within 4 hours. Therefore, performing this method there is no need to wait 24 hours to be sure of the final result as for Marshall test. Furthermore, the wide range of colours from peach through the light green, dark green, light blue and dark blue also provides a semi-quantification of the infection level. In addition, the urease reaction occurs at a pH below 6.2, increasing the specificity of the test because microorganisms other than *H. Pylori,* such as Proteus, Pseudomonas, *E. coli* do not react at acidic pH.

Other tests have been developed: CLO-Test® (Kimberly-Klark, Utah, USA), MRU (J Gastroenterol Hepatol 1992; 7: 569-71), Pronto-Dry® (Eur. J. Gastroenterol. Hepatol 2004 Feb. 16/21 195-9) and RUT (Rapid Urease Test) (S. Afr. Med. J. 2007, 97: 1281-4). However, even in these tests the main limits are essentially related to their non-optimal sensitivity, especially in relation to the need to obtain a sure result in a short time.

In particular, the sensitivity shown corresponds to:
CLO-test: 15% at 5 minutes, 60% at 30 and 88% at 24 hours;
Pronto-Dry 30% at 5 minutes, 50% at 10 minutes and 90% at 60 minutes;
RUT: 70% at 5 minutes, 91% at 24 hours;
MRU: 85% at 5 minutes, 92% at 30 minutes. Therefore, also the response times by these tests, even if lower than the previous, do not ensure an outpatient executability.

In particular, the disadvantages identified in the aforementioned methods can be summarized in the following points:

### 1) Marshall Test

Biopsies with high urease activity develop shortly red colour around the fragment. However, in presence of poor enzymatic activity, the low amount of ammonia significantly increases the incubation time necessary to obtain the colour change. This drawback is due to the phenol red used as indicator that do not possess a sufficiently acidic color change range (6.9 to 8.4 with Pka 7.9). Performing the test at a pH greater than 6.5 can lead to false positive reactions for the presence of different bacteria from *Helicobacter Pylori* (Proteus, Pseudomonas, *E. coli*). These aspects reduce the sensitivity and specificity of the test besides increasing the incubation time required.

### 2) Jackson Test:

The spectrum of colours, secondary to pH changes, is not always well-differentiable, especially at the intermediate pH values between 5.5-7.0, this feature makes the outcome uncertain (light green: negative; dark green: positive) in the presence of poor urease activity.

The available tests should be further improved, increasing their sensitivity, reducing the measurement time and the possibility of false negative results, especially taking into account the factors which may cause false negative results, such as:
- fragmented distribution of the infection: the disease may have a patchy distribution, in order to avoid false negative results more gastric biopsies should be withdrawn and analyzed;
- intestinal metaplasia: *Helicobacter Pylori* does not colonize the intestinal metaplasia zones, therefore the widespread presence of these areas can result in a false negative result; also in this case it would be appropriate to perform multiple mucosa biopsies;
- taking antibiotics and antisecretory: *Helicobacter Pylori* is sensitive to this medicaments intake, which may inhibit but does not destroy the infection;
- choice of the most suitable pH indicators.

The pH indicators are weak acids or bases changing their molecular structure depending on the pH of the solution, with resulting change in colour. To obtain a reasonable sensitivity, the indicator should change colour from pale, as the yellow of non dissociated form, to strong, such as red or blue of the dissociated form. The point of colour change corresponds to the situation wherein the amount of the undissociated form is equivalent to that of the dissociated form (pKa=pH) and the indicator takes on an intermediate colour. Indeed, the point of colour change is sufficiently wide to be considered as a colour change range including a range of values pH=pKa ± 1. Thus, the indicator of choice should have a Pka little above the initial pH of the solution.

The Italian patent application ITRM20030399 discloses a composition to be used in the diagnostic test exploiting urease in the form of aqueous solution able to quickly reveal the presence of *Helicobacter Pylori* in bioptic fragments. The invention provides a totally ambulatory use test solution as its increased sensitivity has significantly shortened the response time. The composition contains 10% urea in deionized water at pH 6.8 and at least one pH indicator having a pKa ranging between 6.0 and 7.0 and at a concentration such to determine a final pH of about 5. However, also this test has certain limitations: beside a great sensitivity in 5 minutes reaction time (100%) in case of high-moderate bacterial load, for low bacterial loads, only 55% of the test samples react within 5 minutes and 90% changes colour in 30 minutes. Furthermore, the use of two different indicators not always provides easy reading outcomes for subtle colour changing at intermediate pH values (7.0 to 7.9) close to the reactants pKa, sometimes making questionable interpretation of the results in case of low infection by *H. Pylori* bacteria. Also, 10% urea does not ensure a sufficiently acid pH suitable for long-term storage of the diagnostic reagent.

The US patent application US 5,702,911 relates to an improved test composition for diagnosing gastric ulcers, duodenal ulcers, gastritis, gastric lymphoma and gastric carcinoma by the detection of Helicobacter pylori and the enzyme catalase associated with such conditions. The test composition is an aqueous solution containing about 0.5% to about 6% hydrogen peroxide, about 0.5% to about 2.0% urea, about 0.001% to about 0.01% monobasic sodium phosphate and about 0.01% to about 0.1% bromothymol blue as indicator. Bhasin D.K. et al. in "Relative merits of various rapid biopsy urease tests for diagnosis of Helicobacter pylori (Campylobacter pylori) J Assoc Physicians India 38 Suppl 1, 689-691, discloses a method for fast detection of Helicobacter pylori which employs 5% urea and an unknown amount of bromothymol blue as indicator.

It should be also considered that, in recent years, the widespread use of generic antibiotic therapies, has increased the detection of low *H. Pylori* load further reducing the sensitivity of the tests.

So, it is still strongly felt the need to develop new reagents and methods to routinely detect the presence of *Helicobacter Pylori* in biopsies during gastroscopy, enabling costs and execution times reduction, alternative to traditional diagnostic techniques (histology, culture, urea breath-test), carried out on gastric mucosa tissue biopsies using improved tests based on the urease activity.

### Summary of the invention

The primary objective of the present invention is to provide an improved composition for diagnostic use enabling to detect in a quick and reliable way the presence of *H. Pylori* in biopsy fragments, even in case of low bacterial load.

The technical problem of providing an improved composition for a diagnostic test enabling to detect quickly and reliably the presence of *H. Pylori* in biopsy fragments, even in the presence of a low bacterial load, is solved by the composition of the present invention characterized by the fact to comprise 5% of urea and bromothymol blue in saline.

It has been surprisingly found that significantly reducing the amount of urea present in the diagnostic composition compared to state of the art compositions, the diagnostic test improves its performance in terms of sensitivity and specificity, it stabilizes the pH and allows the detection, in a quicker and more reliable way, the presence of bacterial infection basing gastric ulcer and duodenal ulcer.

### Detailed description of the invention

It has to be noted that for the purposes of the present invention the expressions: diagnostic composition, diagnostic reagent, enzyme substrate are used herein as synonyms.

A first aim of this invention is to provide a composition in the form of aqueous solution to perform the diagnostic test enabling to rapidly detect, during the gastroscopy analysis, the presence of *Helicobacter Pylori* in gastric mucosa biopsies, comprising 5% urea and a pH indicator having Pka equal to 7.0 in physiological solution (0.9% sodium chloride).

The value of the final pH of the composition according to the invention of about 4.8.

According to the invention the pH indicator is bromothymol blue, an organic compound with weak acid properties, in its normal form (acid) is yellow, whereas its conjugate base is blue. Due to this colour difference between the two forms, bromothymol blue is used as a pH indicator. Furthermore, the bromothymol blue used in the composition according to the invention has a suitable range of pH values for the change of colour (in the yellow-green-blue range) compliant to the presence of ammonia produced by the reaction, able to discriminate the entity the bacterial load by easily detectable colour change (green = low, blue = intense).

This technical feature is an undoubted advantage compared to the majority of tests available on the market using phenol red as a colour indicator, as the medium to low bacterial loads, currently the most frequent, determine a slow colour change, often not well defined. This is because the colour change from yellow (negative) to deep red (positive), passing through the pink shades, results in an uncertain discrimination between the very light pink of the biopsy and a pink due to weak positivity.

According to the invention the amount of bromothymol blue in the composition ranges between 0.5% and 10%. Preferably, the amount of bromothymol blue in the composition ranges between 2% and 6%, in a particularly preferred embodiment of the invention the amount of bromothymol blue in the composition is 4%. The diagnostic composition according to the present invention is very simple, does not requires inclusion of preservatives, antimicrobial agents, or buffering agents. This feature contributes to a degradation reaction of the urea even faster, and consequently, also the speed of detection is maximum. Using the composition of the present invention, the colour changing is observable from the biopsy periphery, the gradation of colour indicates the infectious load present in the sample, allowing the diagnosis already at the end of the endoscopic investigation.

Although the urease reaction that uses the composition of the present invention is carried out optimally at alkaline pH, advantageously the positive reaction begins to emerge from a pH value of less than 6.2. This increases the specificity of the test, provided that several other micro-organisms than *H. Pylori,* such as Proteus, Pseudomonas and *E. coli* can be present in the biopsy piece, but do not react in solution at acidic pH, thus avoiding the possibility of false positive responses.

The composition according to the invention is extremely simple and particularly stable, this enables the production of an analytical system wherein the amount of the diagnostic reagent can be varied and limited to the minimum necessary amount to ensure that the bioptic sample is completely immersed. In such a way, in the reaction medium a high concentration of ammonia is yielded, which causes a rapid increase of the pH value, and thus the quick detection of urease.

In a preferred embodiment of the invention the diagnostic composition is prepared immediately before use by adding to a solution of 0.5%-10% bromothymol blue in physiological solution, more preferably 4% bromothymol blue in physiological solution, the amount of urea required to obtain a final concentration of 5%.

The improved diagnostic composition of the present invention is characterized by a higher sensitivity, and hence specificity, than those of the prior art, so as to provide a better performance of the reaction basing the diagnostic method allowing to use half amount of substrate, i.e. urea. Compared to the use of the composition described in the patent application ITRM20030399, which contains about 10% of urea and two pH indicators, 1% bromothymol blue and 2% phenol red, both detection sensitivity and specificity from the present composition are increased and close to 100%. Moreover, particularly interesting was the surprising effect determined by the particular selection of the solvent medium wherein urea is dissolved, as the bromothymol blue in physiological solution resulted to be more stable and sensitive than deionized water or hydroalcoholic solution used as solvent medium. The use of a water-alcohol solution (60% saline and 40% ethyl alcohol) as solvent medium for the urea powder and the pH indicator, has shown to exert a positive effect on the solubilization of the dye bromothymol blue, but a negative effect on the sensitivity and specificity of the detection reaction especially in those patients using proton pump inhibitors and other drugs whose intake is notoriously not recommended in the days before the endoscopic examination.

In particular, the major improvements resulting from use of the present composition compared to those of the prior art include:
- greater stability of the pH of the ready to use solution;
- maintenance of 100% specificity;
- maintenance of 100% sensitivity within 5 minutes in case of high-moderate bacterial load, increase of sensitivity up to 90% within 5 minutes for low bacterial load;
- better identification of the colour change, hence improved reading for intermediate pH;
- better classification of the infectivity load (low = green at biopsy perifery; moderate = blue at biopsy perifery; high = deep blue spread).

Furthermore, the liquid formulation allows a more rapid diffusion of ammonia resulting from the urease reaction, and the absence of buffers do not reduce the rate of reaction, decreasing the time of reaction of the diagnostic composition.

A further object of the present invention is to provide a composition comprising 5% urea and bromothymol blue ranging between 0.5% and 10%, preferably ranging between 2% and 6%, even more preferably 4% bromothymol blue, in saline (0.9% sodium chloride) having a pH = 4.8 for the use in the rapid and reliable diagnosis of bacteria *Helicobacter Pylori* during gastroscopy.

A further object of the present invention is a diagnostic kit for the ultra-rapid detection of the presence and the bacterial load of *Helicobacter Pylori* in the gastric mucosa biopsy taken during gastroscopy via the evaluation of the activity of urease in biopsy tissue samples comprising:
- a transparent tube with a concave rounded bottom made of polyethylene, or any other plastic material with similar properties, containing 1 ml of 0.5-10 % of bromothymol blue in physiological solution; the tube is equipped with a screw cap, also made of material plastic, having a small tank, closed by means of a septum, containing the amount of powder urea necessary such that the final solution is: 5% urea, 0.5-10 % of bromothymol blue in saline solution (0.9% sodium chloride) with a pH of 4.8;
- two adhesive labels, one for the registration of the data to be affixed externally to the tube, the other one with the specifications of the production batch to be entered on the report to provide the certification of the product used for the detection of *Helicobacter Pylori;*
- leaflet with technical notes and instructions for use.

The diagnostic composition in the form that precedes the use thereof, wherein the urea powder has to be added to the saline solution containing bromothymol blue, that is, when bromothymol blue and urea are still separate, has a shelf life at extreme temperatures of -9 °C and 45 °C of two years; when the two components, the bromothymol blue solution in saline solution and the urea powder, are mixed together the composition is stable for 48 hours at room temperature, and for more than two weeks at +4 °C.

A further object of the present invention is the method for the ultra-rapid detection of the presence and the bacterial load of *Helicobacter Pylori* in the gastric mucosa biopsy during gastroscopy via the evaluation of the activity of urease in the biopsy tissue sample using the diagnostic composition: 5 % urea and 0.5-10% bromothymol blue, preferably 4% bromothymol blue, in physiological solution comprising the steps of:
- contacting the biopsy sample with the diagnostic solution 5% urea and bromothymol blue in physiological saline solution having a pH equal to 4.8, by complete immersion in a ml of solution,
- wait 5 minutes for the colour change to read the outcome.

In a particularly preferred embodiment of the invention the method for the ultra-rapid detection of the presence and the bacterial load of *Helicobacter Pylori* in the gastric mucosa biopsy during gastroscopy via the evaluation of the activity of urease in the bioptic tissue sample is implemented through the use of the diagnostic kit, which is also subject of the invention, using the diagnostic composition comprising 5% urea and 0.5% - 10% bromothymol blue, preferably 4% bromothymol blue, in physiological solution comprising the following steps:
- push the cap of the tube to cause breakage of the septum and then the opening of the internal reservoir containing urea powder, so as the urea powder is dissolved in the bromothymol blue solution;
- shake the tube to mix the components;
- unscrew the cap and deposit one or two pieces of gastric mucosa biopsy in the liquid diagnostic composition, without shaking;
- wait 5 minutes for the colour changing.

In case of very low bacterial loads the test may take 15 minutes.

If the colour of the total solution rapidly spread to the slight green, the liquid may be contaminated with blood or bile present on the surface of the forceps used for taking the biopsy. In this case it is advisable to withdraw the biopsy from the tube and immerge it in a fresh tube.

The use of the diagnostic composition according to the invention and the relative method determines considerable advantages and benefits for both the operator performing the test during gastroscopy and for the patient, and positively influences the diagnosis and therapeutic outcome of the patient.

The use of the diagnostic composition and the relative method thereof according to the invention allows to determine the bacterial load in the gastric mucosa providing evidence of low, medium, high level of infection, even in the event the patient takes proton pump inhibitors and contraindicated drugs in endoscopic examination days.

### Experimental study

EXAMPLE 1 - Clinical study performed by using the diagnostic composition comprising 5% urea, 4% of bromothymol blue in saline according to the invention. A clinical trial was carried out on 318 patients to demonstrate the effectiveness of the composition and the relative method according to the invention, in particular, the study was aimed to demonstrate the high sensitivity of the composition also toward those patients who had taken antisecretory gastric protectors, type lansoprazole, that reduce the enzyme urease activity and make the enzyme reaction much slower to be practised during the gastroscopy or immediately after.

The 318 patients enrolled in the study were divided into two groups. The first group included 255 patients who had taken no antisecretory gastric protectors drugs and 63 patients who had taken antisecretory gastric protectors. To validate the results of the diagnostic test according to the invention, all patients were subjected to analysis of the presence of *H. Pylori* in the same time with three different techniques:
- rapid test on biopsy according to the method of the invention,
- histological examination of biopsy;
- breath test (urea breath test).

The histological examination of biopsy and breath tests (urea breath test) were conducted according to techniques known to experts in the field, while the rapid test on the biopsy was conducted with the composition of the invention. In particular, from the gastric mucosa a biopsy of approximately **1** mm³ size was withdrawn, and placed in the described test tube containing the diagnostic composition of the invention to be analyzed. The diagnostic composition is obtained by mixing the proper amount of urea in the solution of bromothymol blue in order to have a final ready to use solution of 5% urea and 4% of bromothymol blue in physiological saline solution having a pH equal to 4.8.

### Results

The diagnostic test using the composition according to the invention has shown a specificity of 100% and an overall sensitivity of 93%, wherein 100% for intermediate-high *H. Pylori* load and 92% for very low *H. Pylori* load.

Out of the 295 tested patients in the first group who had taken no antisecretory gastric protectors drugs (lansoprazole), 185 patients resulted to be non affected by *Helicobacter Pylori* infection and 110 patient resulted to be affected; the results obtained by the test has been confirmed by the data obtained with the other two diagnostic methods. Nine cases have shown a discrepancy of results because the rapid test was negative, also read after 24 hours, while a low level positive result was shown by both histology and breath test (false negative).

In the second group, 41 biopsies were found without *H. Pylori* and 62 with the bacterial infection. 4 biopsies obtained a doubt result: they were negative, also if read after 24 hours, whereas breath test and/or histological examination were slightly positive.

So out of the 398 patients enrolled, 385 have shown a rapid correlation of the test with at least one of the other assays, therefore, the test has shown 100% specificity and an overall sensibility of 93%.

No difference in sensibility and specificity appeared in both groups with and without antisecretory gastric protectors drugs.

The results of this study demonstrate that the composition and the method according to the invention is a useful tool for the detection of *H. Pylori* which can be run directly during the gastroscopy investigation, with high sensitivity and specificity of outcome equal to 93% and 100% respectivly; particularly useful and advantageous is the high reliability of result even in patients taking antisecretory in the days of gastroscopy.

EXAMPLE 2 - Clinical study performed by using the diagnostic composition containing 10% urea in deionized water at pH 6.8, 1% of bromothymol blue and 2% phenol red.

By using a diagnostic composition containing 10% urea in deionized water at pH 6, 8, 1% of bromothymol blue and 2% phenol red, as described in patent application ITRM20030399, an overall sensitivity of 88% compared to the histological data identified as the standard reference for the diagnosis of presence of the bacterium was obtained. In this case, the study enrolled 86 patients, non-treated with antisecretory gastric protectors drugs; out of the 86 patients, 29 were resulted without *H. Pylori* and 57 resulted to be infected. Out these 57 infected biopsies, 7 had a high bacterial load, 27 presented intermediate load, 23 a low infection level. Out of these 23 biopsy samples with very low bacterial load, 7 have not reacted in the diagnostic test that uses the composition containing 10% urea in deionized water at pH 6.8, 1% of bromothymol blue and 2% phenol red.

## Claims

1. Composition to quickly detect the presence of *Helicobacter Pylori* in biopsy specimens of the gastric mucosa during the gastroscopy analysis **characterized in that** it comprises 5% urea and 0.5-10% of bromothymol blue in saline solution having pH equal to 4.8.

2. Composition according to claim 1 wherein the concentration of bromothymol blue in saline is ranging between 2 and 6%.

3. Composition according to claims 1 or 2 wherein the concentration of bromothymol blue in saline is 4%.

4. Composition comprising 5% urea and 0.5-10% of bromothymol blue in saline solution having pH equal to 4.8 suitable for the use in the diagnosis of the bacterial load of *Helicobacter Pylori* in the biopsy of the gastric mucosa during the gastroscopic analysis.

5. Composition according to claim 4 wherein bromothymol blue in saline solution is 4%.

6. Composition according to claims 4 and 5 suitable for use in a method comprising the following steps:
- contacting the biopsy sample with the composition by complete immersion in a ml of solution,
- waiting 5 minutes for the colour change to read the outcome.

7. Diagnostic kit for the rapid detection of the presence and bacterial load of *Helicobacter Pylori* in gastric mucosa biopsy taken during gastroscopy comprising:
- a transparent tube with a concave rounded bottom made of polyethylene, or any other plastic material with similar properties, containing 1 ml of 0.5-10 % of bromothymol blue in physiological solution; the tube being equipped with a screw cap, also made of material plastic, having a small tank, closed by means of a septum, containing the amount of powder urea necessary such that the final solution is: 5% urea, 0.5-10 % of bromothymol blue in saline solution (0.9% sodium chloride) with a pH of 4.8;
- two adhesive labels, one for the registration of the data to be affixed externally to the tube, the other one with the specifications of the production batch to be entered on the report to provide the certification of the product used for the detection of *Helicobacter Pylori;*
- leaflet with technical notes and instructions for use.

## Patentansprüche

1. Zusammensetzung zur schnellen Feststellung des Vorhandenseins von *Helicobacter pylori* in Biopsieproben der Magenschleimhaut im Zuge der Gastroskopieanalyse, **dadurch gekennzeichnet, dass** sie 5% Harnstoff und 0,5-10% Bromthymolblau in Kochsalzlösung mit einem pH-Wert gleich 4,8 umfasst.

2. Zusammensetzung gemäß Anspruch 1, wobei sich die Konzentration von Bromthymolblau in Kochsalzlösung im Bereich zwischen 2 und 6% bewegt.

3. Zusammensetzung gemäß Ansprüchen 1 oder 2, wobei die Konzentration von Bromthymolblau in der Kochsalzlösung 4% beträgt.

4. Zusammensetzung, die 5% Harnstoff und 0,5-10% Bromthymolblau in der Kochsalzlösung mit einem pH-Wert gleich 4.8 umfasst und die zur Verwendung bei der Diagnose der bakteriellen Belastung durch *Helicobacter pylori* in der Biopsie der Magenschleimhaut im Zuge der Gastroskopieanalyse geeignet ist.

5. Zusammensetzung gemäß Anspruch 4, wobei das Bromthymolblau in der Kochsalzlösung 4% ausmacht.

6. Zusammensetzung gemäß Ansprüchen 4 und 5, die zur Verwendung bei einem Verfahren mit den folgenden Schritten geeignet ist:
- Kontaktieren der Biopsieprobe mit der Zusammensetzung durch vollständiges Eintauchen in einem ml Lösung,
- 5-minütiges Warten, bis die Farbveränderung das Ergebnis anzeigt.

7. Diagnostisches Kit zur schnellen Feststellung des Vorhandenseins von und der bakteriellen Belastung durch *Helicobacter pylori* bei einer Magenschleimhaut-Biopsie, welche im Zuge einer Gastroskopie entnommen wird, umfassend:
- ein transparentes Rohr mit einer konkaven, abgerundeten Unterseite, welche aus Polyethylen oder einem beliebigen anderen Kunststoffmaterial mit ähnlichen Eigenschaften besteht, welches 1 ml von 0,5-10% Bromthymolblau in einer physiologischen Lösung enthält; wobei das Rohr mit einem Schraubverschluss ausgestattet ist, der ebenfalls aus einem Kunststoffmaterial besteht und einen kleinen Tank aufweist, der mittels eines Septums verschlossen wird und eine notwendige Menge an Harnstoffpulver enthält, so dass die endgültige Lösung wie folgt ist: 5% Harnstoff, 0,5-10% Bromthymolblau in Kochsalzlösung (0,9% Natriumchlorid) mit einem pH-Wert von 4.8;
- zwei Klebeetiketten, eine für die Registrierung der Daten zum externen Anbringen an dem Rohr, die andere mit den Spezifikationen für die Produktionscharge zur Angabe im Bericht, um die Zertifizierung des Produkts bereitzustellen, das zur Feststellung von *Helicobacter pylori* verwendet wird;
- Merkblatt mit technischen Hinweisen sowie Gebrauchsanweisungen.

## Revendications

1. Composition pour détecter rapidement la présence d'Helicobacter Pylori dans des échantillons de biopsie de la muqueuse gastrique durant l'analyse de gastroscopie, **caractérisée en ce qu'**elle comprend 5% d'urée et 0,5-10% de bleu de bromothymol en solution saline ayant un pH égal à 4,8.

2. Composition selon la revendication 1, dans laquelle la concentration de bleu de bromothymol en solution saline est dans la plage entre 2 et 6%.

3. Composition selon la revendication 1 ou 2, dans laquelle la concentration de bleu de bromothymol en solution saline est de 4%.

4. Composition comprenant 5% d'urée et 0,5-10% de bleu de bromothymol en solution saline ayant un pH égal à 4,8, adaptée pour l'utilisation dans le diagnostic de la charge bactérienne d'Helicobacter Pylori dans la biopsie de la muqueuse gastrique durant l'analyse de gastroscopie.

5. Composition selon la revendication 4, dans laquelle le bleu de bromothymol en solution saline est 4%.

6. Composition selon les revendications 4 et 5, adaptée pour l'utilisation dans un procédé comprenant les étapes suivantes :
- la mise en contact de l'échantillon de biopsie avec la composition par immersion totale dans un ml de solution,
- l'attente de 5 minutes pour le changement de couleur pour lire le résultat.

7. Kit de diagnostic pour la détection rapide de la présence et la charge bactérienne d'Helicobacter Pylori dans une biopsie de muqueuse gastrique prélevée durant une analyse de gastroscopie, comprenant :
- un tube transparent avec un fond arrondi concave réalisé en polyéthylène, ou n'importe quel autre matériau plastique avec des propriétés similaires, contenant 1 ml de 0,5-10% de bleu de bromothymol en solution physiologique ; le type étant équipé d'un bouchon vissé, également réalisé en matériau plastique, ayant un petit réservoir, fermé au moyen d'une cloison, contenant la quantité d'urée en poudre nécessaire pour que la solution finale soit : 5% d'urée, 0,5-10% de bleu de bromothymol en solution saline (0,9% de chlorure de sodium) avec un pH de 4,8 ;
- deux étiquettes adhésives, une pour l'enregistrement des données à appliquer à l'extérieur du tube, l'autre avec les spécifications du lot de production à saisir sur le rapport pour fournir la certification du produit utilisé pour la détection d'Helicobacter Pylori ;
- un dépliant avec des notes techniques et des instructions pour l'utilisation.
